# EUROPEAN PATENT APPLICATION

(11) **EP 1 967 149 A1**
(43) Date of publication of application: **10.09.2008**
(21) Application number: 06769543.7
(22) Date of filing: 13.06.2006
(51) Int. Cl.: A61B 17/66

(54) **COMPRESSION-DISTRACTION DEVICE**

(30) Priority: 29.12.2005 RU 2005141637
(71) Applicant: Federalnoe Gosudarstvennoe Uchrezhdenie Rossiisky Nauchny Tsentr "Vosstanovitelnaya Travmotologiya I Ortopediya", Kurgan 640014 (RU)
(72) Inventor: MATSUKIDIS, Theodoros, 563 34 Thessaloniki (GR); SHEVTSOV, Vladimir Ivanovich, Kurgan 640020 (RU)
(74) Representative: Sloboshanin, Sergej
(86) International application number: PCT/RU2006/000302
(87) International publication number: WO 2007/075114

(57) **Abstract**

The invention relates to medicine, in particular to traumatology and orthopaedics and can be used for treating patients, whose members are damaged or diseased, in particular for removing deformations and shortening of limbs. The inventive device comprises supports (1, 2) embodied in the form of demountable rings (3, 4), which are coaxially arranged with respect to each other and one of which (3) is embodies in the form of the internal ring provided with a ring gear (21) arranged on the external end surface thereof and the other ring (4) is embodied in the form of an internal ring (4) provided with external eyes (23) which are provided with holes (24) and used for connecting the supports (1, 2). Said device also comprises threaded rods (25, 26, 27, 28), repositioning units (29, 30), bone fixtures (69) and a locking element (63). Said threaded rods (25, 26, 27, 28) are pairwisely and hingedly fixed to threaded bars, which are arranged in a parallel position to each other, brought into contact with the repositioning units (29, 30), and located in sockets (31) which are rigidly fixed to the eyes (23) of the external rings (4), wherein each repositioning units (29, 30) is embodied in the form of a L-shaped (46) or provided with holes (57) for the threaded bar (45) slide block, a locking element is embodied in the form of a bolt (63) provided with a supporting area (64) on the head base and with teeth (65), which are positioned along the leg circumference and are used for inserting the eyes (23) into the hole (24) which communicates with a groove (22) in such a way that it contacts the teeth of the ring gear (21), washers (66) provided with flanges (67) and positioned along the hole circumference and a nut (68). The unitised configuration of the device makes it possible to reduce the mounting time thereof, to simplify handling operations and to increase the correction accuracy of the position of bones and the chips thereof.

## Description

### Field of the Invention

The invention relates to the medical field, particularly to traumatology and orthopedics, and may be utilized for the treating of patients with bone injuries and diseases of extremities.

### Prior Art

Known an apparatus for transosseous osteosynthesis of Ilizarov, comprising supports held by the telescopic screws and the reposition units disposed between them, shaped as the interconnected support elements and tie bars (A.C. Nº 1055499 USSR, Published 23.11.1983, Bulletin Nº 43).

However using this apparatus for reducing multi-component, including rotary deformations of the extremity segments needs the mounting of the multi-detail functional units, it makes difficult to realize the osteosynthesis, in some cases it needs the repeated mounting of the apparatus.

Also known a compression-distraction device, comprising supports in the form of sectional, coaxially disposed relatively to each other rings, one of them is inner with a ring gear on the external butt, another - external with lugs supplied with holes, and also rods, reposition units, bone fixators fixed on the inner rings, removable key with a star-shaped head, locking element and fixation elements (A.C. Nº 1132934 (USSR), Published 07.01.1985, Bulletin Nº 1).

However this device is mainly intended for the length reconstruction of the segments of extremities, and also for the reposition of fragments at fractures with slight displacements. This device in view of its constructive features can't be efficiently used for the reducing of the complex multi-component deformations. It's caused both by the connection of supports schema and the reposition units construction which don't provide the prevention of the repeated displacement of the fragments during the reposition. Besides, in the construction under consideration the reducing of one kind of fragments displacement needs to make some consistent or synchronous manipulations, which makes difficult the exploitation of the apparatus.

### Disclosure of the Invention

The object of the invention is to design the construction of the compression-distraction apparatus, which would allow to simplify its exploitation and to extend the functional possibilities at the treating of patients with the shortenings of the segments of extremity, combining with the multi-component deformation.

Indicated object is accomplished due to the fact that in the compression-distraction apparatus comprising the supports in the form of sectional, coaxially disposed relatively to each other rings, one of them is inner with a ring gear on the external butt, another - external with lugs supplied with holes, threaded rods, reposition units, bone fixators held on the inner rings, locking element and fixing elements in the form of washers, nuts and fixing bolts, the external ring is formed with the slot for the ring gear on the inner butt, the holes in the lugs are intended for the threaded rods, but, at least one of these holes is communicated with the slot on the inner butt - for the locking element, the component inner rings of the sector having the holes on the ends, commensurable with the slot and the jut, and the external ones - are shaped as a L-reversed and commensurable with the rectangular slot on the external side, with the hollow for the thread bushing - on the inner side, the groove which is coaxial with the hole of bushing - on the butt, at that the fixing clamp is placed on the lasts, it is supplied by juts on the ends, commensurable with slots on the external surface of the curves of ends shaped as a L-reversed of the sectors of external rings, and the groove formed for the fixing bolt on the inner surface of the butt with the circular flute in the base of head, threaded rods are hinged in pairs on the threaded bars parallely placed and getting in touch with the reposition units, and placed in the cartridges with a flank hollow for the nut and the threaded hole for the locking bolt contacting with nut on one of the ends, and the other end is firmly fixed in the lugs of the external rings, each of the reposition units is shaped as a L-reversed slider supplied with the holes for the threaded bar, the frame of which is supplied with juts for the rectangular slots on the external surface of the sectors ends of external rings and the hole for the locking bolt - in the base, and also the groove for the fixing bolt with the circular flute on its stem ― on the butt of the base, the locking element is in the form of the bolt with the support plate in the base of head and with cogs - along the perimeter of its stem for placing lugs in the hole, communicant with the slot, providing the contact with the ring gear, and also washers with the pad - along the perimeter of the hole and the nuts.

It's also foreseen that:
- the threaded rods are connected with the threaded bars by the jaws, hinged on the bushings placed on the threaded bars with the displacement possibility;
- the cartridges are supplied with the external thread, the sector hollow on one of the ends and secured in the lugs of the external rings by means of the washer with the sector jut conforming to the hollow on the end of the cartridge and the nut;
- the cartridges are supplied with the inner thread on one of the ends and secured in the lugs of the external rings by the ducted bolt with the sector hollow on the threaded stem and the washer with the sector jut conforming to the hollow on the stem of the bolt;
- the slider of the reposition unit is sectional, comprising the base with the figured slots on the flank sides and the removable bracket fixed on it; the bracket has the holes for the threaded bar with stems in its base conforming to the figured slots, and the holes for the fixing bolt.

The compression-distraction apparatus is explained by the description and the schemes, which represent:
Fig. 1 - the compression-distraction apparatus, a general view;
Fig. 2 - the inner and the external rings of supports, the scheme of their connection;
Fig. 3 - the sectors of the external ring of support, the scheme of their connection;
Fig. 4 - the sectors of different corners, the scheme of their connection at the open-ended supports mounting;
Fig. 5 - the threaded rod, the cartridge with the thread on the end and the scheme of its fixing to the support;
Fig. 6 - the threaded rod, the cartridge and the scheme of its fixing to the support by the ducted bolt;
Fig. 7 - the cartridges with the threaded rods inside of them and the scheme of connecting at their fixing to the intermediate support;
Fig. 8 - the slider in the form of the L-reversed and the scheme of its placing;
Fig. 9 - the sectional slider in the form of the L-reversed and the scheme of its assembling;
Fig. 10 - the locking element and the scheme of its mounting.

### Preferred embodiments of the Invention

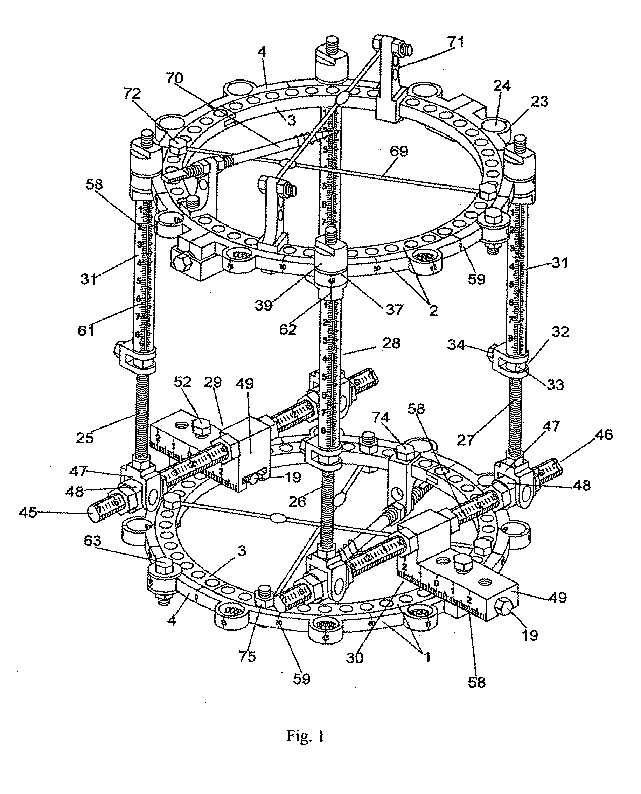

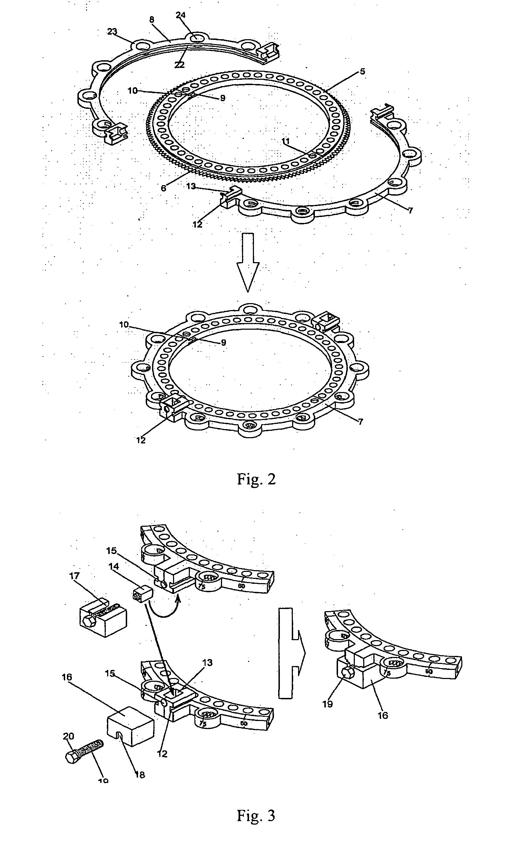

The compression-distraction apparatus (Fig. 1) consists of the supports 1 and 2 in the form of sectional, coaxially placed, with the displacement possibility relatively to each other, the inner - 3, and the external rings - 4, comprising, accordingly, the sectors 5, 6, 7, 8 (Fig. 2). The sectors 5,6 of the inner ring 3 are interconnected on their ends by the commensurable slots 9 and the juts 10 with holes 11. The ends of sectors 7, 8 of the external ring 4 are shaped as a L-reversed, with rectangular slots 12 - on the external side, commensurable with the hollow 13 for the thread bushing 14 - on the inner side and the groove 15 which is coaxial with the hole of bushing 14 for the fixing bolt - on the butt, and are secured by the fixing clamp 16 (Fig. 3) placed on them, the clamp is supplied with the juts 17 on the ends, conforming to the slots 12 on the external surface of the sectors, and the groove 18 formed for the fixing bolt 19 with the circular flute 20 in the base of head - on the inner surface of the butt. The inner rings 3 are supplied with the ring gear 21 on the external butt, and the external rings 4 - by the slot 22 for the ring gear 21 - on the inner and the external butt - with lugs 23, holes 24, at least one of them is communicated with the slot 22 (Fig. 2).

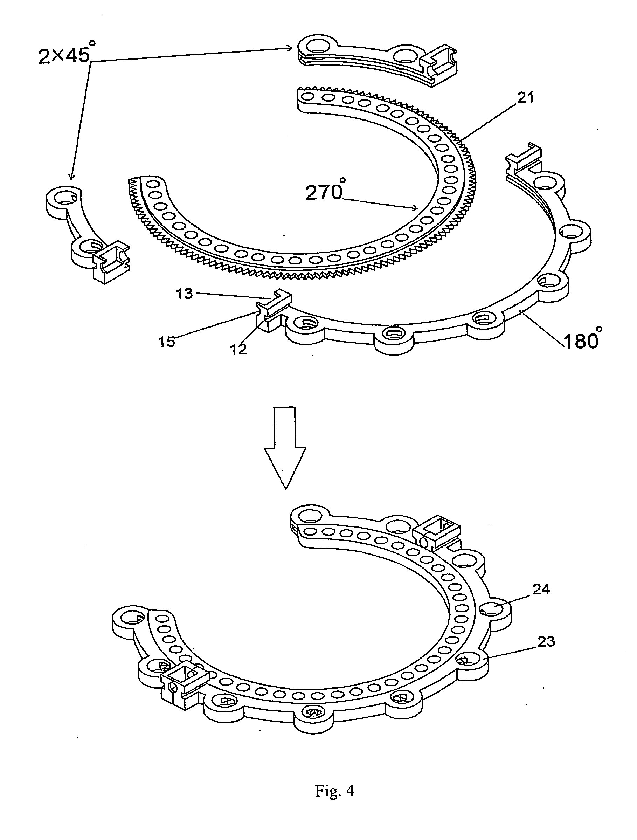

It's foreseen that the sectors 5, 6, 7 and 8 might be of different corners size, and supports 1, 2 ― open-ended (Fig. 4).

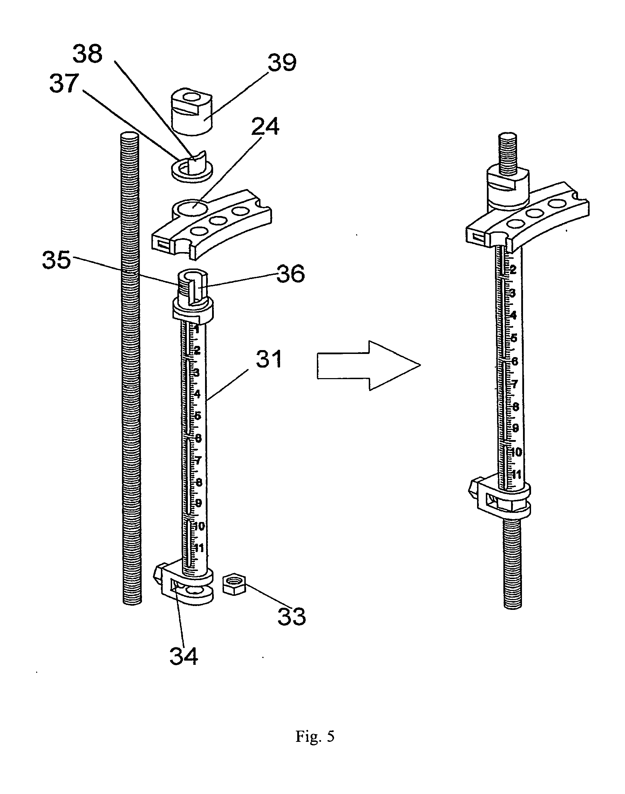

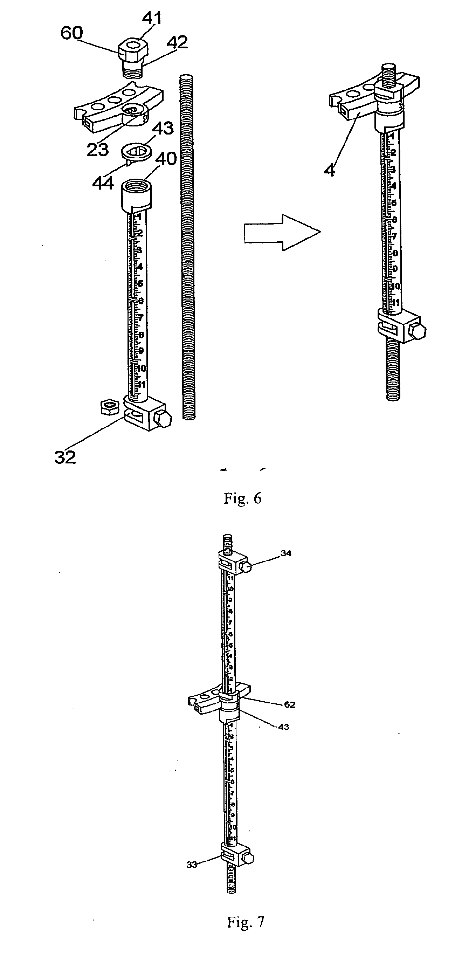

Supports 1, 2 are interconnected by the threaded rods 25, 26, 27, 28 and the reposition units 29, 30. At that mentioned threaded rods with the displacement possibility are placed into the cartridges 31 (Fig. 5) having the flank hollow 32 for the moving nut 33 and the threaded hole for the contacting locking bolt 34 on one of the ends; on the other end ― the external thread 35 and the sector hollow 36, by means of which and also of the washer 37 with the sector jut 38 conforming to the hollow 36, and the nut 39 the threaded rods are firmly fixed in the holes 24 of lugs 23 of the external rings 4 of supports 1, 2. It's also foreseen that the fixing end of the cartridge 31 (Fig. 6) might be supplied with the inner thread 40 and firmly fixed in the holes 24 of lug 23 of the external ring 4 by the ducted bolt 41 with the sector hollow 42 on the threaded stem and by the washer 43 with the sector jut 44 conforming to the hollow 42.

At the same time, the commensurable ends might interconnect the cartridges 31 with the inner thread 40 on one of the ends and the cartridges with the external thread 35 and the sector hollow 36 on one of the ends by means of the washer 37 with the sector jut 38, at the fixing of the intermediate support in the holes 24 of the lugs 23.

The threaded rods 25, 26, 27, 28 placed into the cartridges 31 are fixed in pairs on the parallely placed threaded bars 45, 46 interconnecting with the reposition units 29, 30 by means of the jaws 47 hinged with the bushings 48 placed on the bars with displacement possibility.

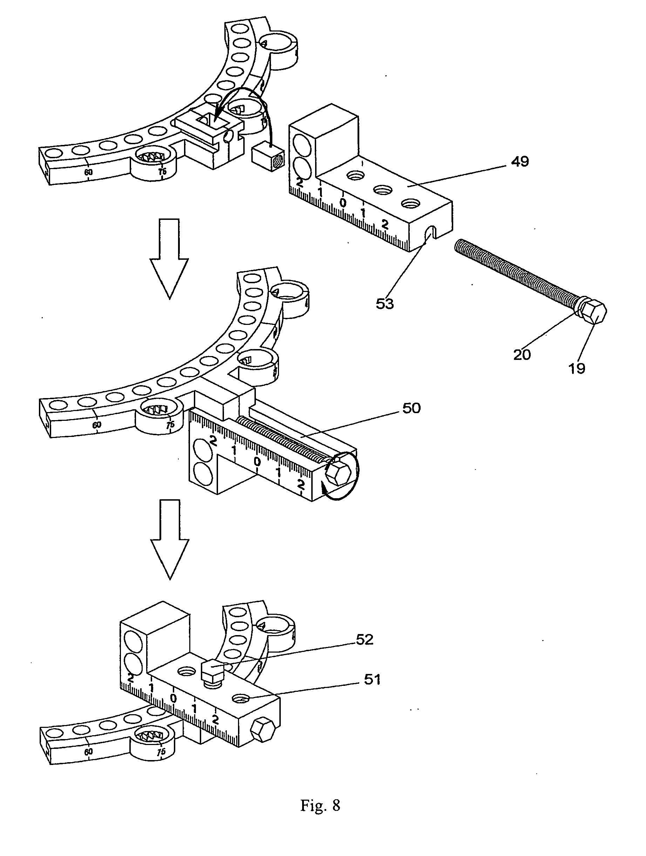

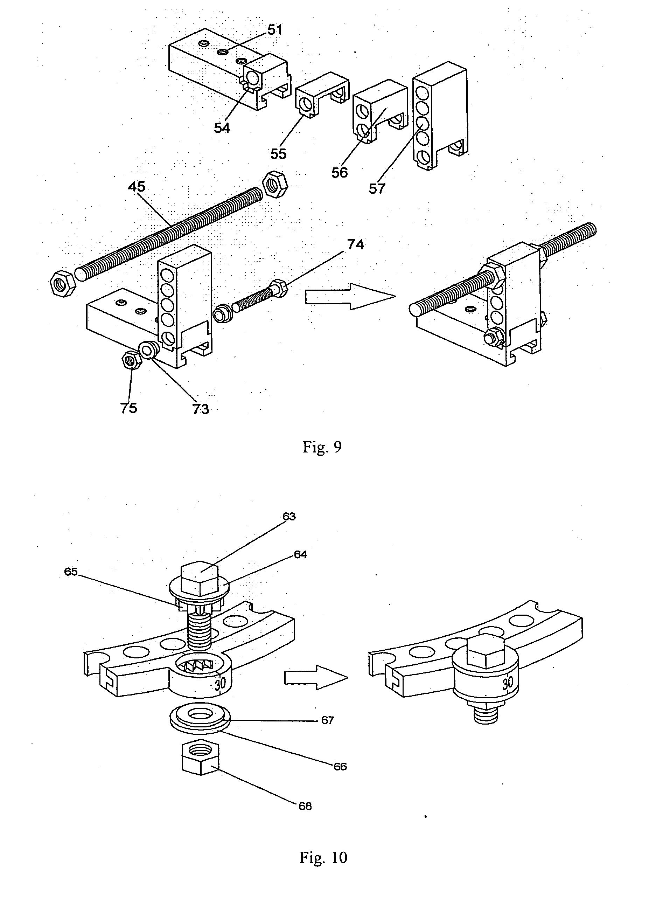

Each of the reposition units 29, 30 is shaped as a L-reversed slider 49 (Fig. 8), the frame of which has the holes for the threaded bars 45, 46, the juts 50 and the holes 51 for the locking bolt 52 - in the base of the frame, and also the groove 53 ― on the butt of the base. It's also foreseen that the slider 49 is sectional (Fig. 9) and supplied with the figured hollows 54 on the flank sides of the base conforming to the stems 55 of the removable bracket 56 with holes 57 for the threaded bars 45, 46. The slider 49 with the displacement possibility is placed in the rectangular slots 12 on the external surface of the curves shaped as a L-reversed of the confronted ends of sectors 7, 8 of external rings 4 and fixed by the locking bolt 52 and the fixing bolt 19 with the circular flute 20, let pass through the groove 53, confronted grooves 15 and put into the thread bushing 14 situated in the confronted hollows 13.

It's also foreseen that the cartridges 31, threaded bars 45, 46 and sliders 49 are supplied with a metric scale 58, a degree scale 59 on the butt of the external ring 4, and a landmark sign 60 - on the surface of the ducted bolt 41. Besides, the cartridges 31 have the longitudinal through slots 61 and the landmark sign 62 - on the surface of the end fixed on the support (Fig. 1, 3, 5, 6, 8).

The apparatus is provided with the locking element (Fig. 10) in the form of the bolt 63 with the support plate 64 in the base of head and with cogs 65 along the perimeter of its stem and also washers 66 with the pad 67 along the perimeter of the hole and the nut for the stabilization and the rotary displacement of the rings 3, 4 of supports 1, 2 relatively to each other.

The bone fixators ― the pins 69 are directly secured by the bolts-fixators 72, the rods 70 - indirectly, by the brackets 71 on the inner rings 3 of supports 1, 2.

Some details and units of the apparatus are interconnected by means of washers 73, fixing bolts 74 and nuts 75.

The compression-distraction apparatus is used as follows.

After the transosseous or cantilever introduction of the bone fixators - the pins 69 are directly secured by the bolts-fixators 72, the rods 70 ― indirectly, by the brackets 71 on the inner rings 3 of supports 1, 2.

Then the supports 1, 2 are interconnected by the confronted ends of sectors 7, 8 fixed on the curves shaped as a L-reversed of the external rings 4, the reposition units 29, 30 and the threaded rods 25, 26, 27 and 28 placed in the cartridges 31.

For the fixing of cartridges 31 having the external thread 35 and the sector hollow 36 on one of the ends, so this end is introduced into the hole 24 of lug 23 of the external rings 4 of supports 1 and 2; the washer 37 with the sector jut 38 is placed on this end and secured by the nut 39. In case of using of the cartridge 31, the end of which has the inner thread 40, and is put in the hole 24 of lug 23 of the external ring 4; the ducted bolt 41 is screwed in the hole 24; the ducted bolt 41 has the sector hollow 42 and the washer 43 with the sector jut 44 conforming to the hollow 42 preliminary placed on the threaded stem. At the coaxial connecting of two cartridges 31, as during of their fixing on the intermediate support of the apparatus, the end of one of the cartridges 31 is introduced into the hole 24 of lug 23; for example, the end of the cartridge 31 having the external thread 35 with the sector hollow 36 and the washer 37 with the sector jut 38 preliminary placed on it is screwed in the cartridge with the inner thread 40. In all cases the mounting of cartridges is corrected from the landmark signs 60 and 62 on the ducted bolts 41 and on the ends of cartridges 31 with the external thread; the landmark signs 60 and 62 must be confronted to the sign of the degree scale 59, marked on the lug 23 of the external ring 4.

By-turn, the threaded rods 25, 26, 27 and 28 situated in the cartridges 31 are fixed in pairs on the parallely placed threaded bars 45, 46 interconnecting with the reposition units 29, 30. The fixing is made up by means of the jaws 47 hinged to the bushings 48 with displacement possibility, which are placed on the mentioned bars. The position of the rods 25, 26, 27 and 28 is fixed by means of the locking bolt 34 contacting with the moving nut 33 mounted on the rod and placed in the hollows 32 of the cartridges 31.

The longitudinal (axial) displacement of the supports 1, 2 and, consequently, of the bone fragments fixed by the pins 69 or by the rods 70, needs to loosen the pull of the threaded rods 34 fixing the position of the nuts 33 in the flank hollows 32 on the ends of cartridges 31. Then the nuts 33 are to be equally rotated in a one or another direction with the consistent displacement of the threaded rods 25, 26, 27 and 28, which results the even distraction or compression of the bone fragments.

The angle displacement of the supports needs the dissymmetry rotation of the moving nuts 33 with the displacement of the rods, for example, the rods 25 and 27 placed in the concave side of the deformation ― in a one direction, accordingly, the rods 26 and 28 placed in the convex side of the deformation - in a opposite direction. The simultaneous lengthening and reducing of the angle deformation of the segment needs the rotation of the moving nuts 33 in a one direction, but the rods 25 and 27 placed in the concave side of the deformation - on the larger size, and the rods 26 and 28 placed in the convex side of the deformation - on the smaller size.

The lateral displacements ("on width" in the deformation plane and in its perpendicular plane) are made up by means of reposition units 29, 30. For that the fixing nuts 75 stabilizing the position of the threaded bars 45, 46 or the bolt 19 fixing the position of the slider 49 are rotated in a one or another direction.

The locking element is used for the displacement of the supports relatively to each other. For that it's necessary to loosen the pull of the bolt 63 fitted in the hole 24 of the lug 23, contacting to the cogs 65 of the ring gear 21 with its turning in the needed direction and to realize the rotation of the supports. Then the washer 66 is screwed and tightened on the stem end of the bolt 63 jutting of the opposite side of the hole 24, the pad 67 conforming to the perimeter of the hole of washer is introduced to the hole 24, the fixing nut 75 is tightened to stabilize the position of the rings 3, 4. All kinds of displacement of the fixing bone fragments can be reduced by the combination or the modification of the consecution of above-mentioned manipulations.

The displacement size of the supports is visually controlled with the help of the metric and degree scales 58, 59, accordingly, on the cartridges 31, the bars 45, 46, the butts of the external rings 4, and also with the help of the through slots 61 on the cartridges 31.

After the dosed displacement of the fragments by means of fixing nuts 75 and bolts 74 the position of some details and units of the apparatus are to be stabilize; the following demounting is effected in the reverse sequence.

### Industrial Applicability

The proposed apparatus is simple in use and has many functional possibilities for the treatment of patients with shortenings of the segments of extremity, combining with its multi-component deformation. The minimum of details composing the uniform assembling makes possible to reduce the time of the apparatus mounting for all kinds of deformations and displacements of the bone fragments; to simplify the manipulations, to raise the exactness of the correction; to make easier the postoperative management of patients, to refuse of the periodical apparatus remounting. At the same time, the mentioned advantages allow using this construction with electron-digital devices with software, for the osteosynthesis in the automatic mode.

## Claims

1. The compression-distraction apparatus comprising supports in the form of sectional, coaxially disposed relatively to each other rings, one of them is inner with a ring gear on the external butt, another ― external with lugs having holes, threaded rods, reposition units, bone fixators, locking element and fixing elements in the form of washers, nuts and fixing bolts, ***characterized* in that** the external ring has the slot the inner butt for the ring gear, the holes in the lugs are intended for the threaded rods, but, at least one of these holes is communicated with the slot on the inner butt - for the locking element, the component inner rings of the sector have the holes on the ends, commensurable with the slot and the jut, and the external ones - are shaped as a L-reversed and commensurable with the rectangular slot on the external side, with the hollow for the thread bushing - on the inner side, the groove which is coaxial with the hole of bushing - on the butt, at that the fixing clamp is placed on the lasts, having the juts on the ends, commensurable with slots on the external surface of the curves of the ends of sectors shaped as a L-reversed of the external rings, and the groove formed for the fixing bolt on the inner surface of the butt with the circular flute in the base of its head, connecting supports, threaded .rods are hinged in pairs on the threaded bars parallely placed and getting in touch with the reposition units and placed in the cartridges with a flank hollow for the nut and the threaded hole for the locking bolt contacting with nut on one of the ends, and the other end is firmly fixed in the lugs of the external rings, each of the reposition units is shaped as a L-reversed slider supplied with holes for the threaded bar, the frame of which has the juts for the rectangular slots on the external surface of the ends of sectors of the external rings and the hole for the locking bolt - in the base, and also the groove for the fixing bolt with the circular hollow on its stem - on the butt of the base, the locking element is in the form of the bolt with the support plate in the base of head and with cogs along the perimeter of its stem for placing lugs in the hole, communicant with the slot, providing the contact with' the ring gear, and also washers with the pad along the perimeter of the hole and the nuts.

2. The compression-distraction apparatus as claimed in the claim 1. ***characterized* in that** the threaded rods are connected with the threaded bars by the jaws, hinged on the bushings, placed on the threaded bars with the displacement possibility.

3. The compression-distraction apparatus as claimed in the claim 1. ***characterized* in that** the cartridges are supplied with the external thread, the sector hollow on one of the ends and secured in the lugs of the external rings by the washer with the sector jut conforming to the hollow on the end of the cartridge and the nut.

4. The compression-distraction apparatus as claimed in the claim 1. ***characterized* in that** the cartridges are supplied with the inner thread on one of the ends and secured in the lugs of the external rings by the ducted bolt with the sector hollow on the threaded stem and the washer with the sector jut conforming to the hollow on the stem of the bolt.

5. The compression-distraction apparatus as claimed in the claim 1. ***characterized* in that** the slider of the reposition unit is sectional, comprising the base with the figured slots on the flank sides and the removable bracket fixed on it; the bracket has the holes for the threaded bar with stems in its base conforming to the figured slots, the holes for the fixing bolt.
